# EUROPÄISCHE PATENTANMELDUNG

(11) **EP 4 151 240 A1**
(43) Veröffentlichungstag der Anmeldung: **22.03.2023**
(21) Anmeldenummer: 22178862.3
(22) Anmeldetag: 14.06.2022
(51) Int. Cl.: A61L 2/00, B05B 12/08, B05B 7/32, B05B 15/40, A62C 35/00, A01G 25/00

(54) **SPRINKLERANLAGE**

(30) Priorität: 20.09.2021 DE 102021124251
(71) Anmelder: Lammers, Bernhard Johannes, 46414 Rhede (DE)
(72) Erfinder: Lammers, Bernhard Johannes, 46414 Rhede (DE)
(74) Vertreter: Dr. Binder & Binder GbR

(57) **Zusammenfassung**

Es wird eine Sprinkleranlage (1) vorgestellt, die ein Strömungsleitungssystem mit einem Wasseranschluss (2), mehreren Fluidleitungen, mindestens einer Pumpe (3) und mehrere, in einem Raum verteilt angeordnete Spritzdüsen (4) zur Zerstäubung einer über die Spritzdüsen (4) abzugebenden Flüssigkeit (5) umfasst.

Die Erfindung ist dadurch gekennzeichnet, dass mindestens ein Vorratsbehälter (6, 7, 8) mit einem flüssigen Zuschlagstoff (9, 10, 11) zur Herstellung eines Gemisches mit Wasser vorhanden ist, in den saugseitig eine druckseitig mit einem Verteilerrohr (12) gekoppelte Saugleitung (13, 14, 15) mündet, wobei diese Saugleitung (13, 14, 15) eine durch eine elektronische Steuerungsvorrichtung (16) ansteuerbare Dosierpumpe (17, 18, 19) zur Förderung des Zuschlagstoffes (9, 10, 11) aufweist.

## Beschreibung

Die Erfindung betrifft eine Sprinkleranlage nach dem Oberbegriff des Patentanspruches 1.

Eine derartige Sprinkleranlage umfasst in an sich bekannter Weise ein Strömungsleitungssystem mit einem Wasseranschluss, mehrere Fluidleitungen, mindestens eine Pumpe und mehrere, in einem Raum verteilt angeordnete Spritzdüsen zur Zerstäubung einer über die Spritzdüsen abzugebenden Flüssigkeit, bei der es sich bei den bislang bekannten Sprinkleranlagen ausschließlich um Wasser handelt. Zum Einsatz kommen derartige Sprinkleranlagen nicht nur zur Brandbekämpfung. Vielmehr können sie auch eingesetzt werden, um beispielsweise eine Bewässerung von Gartenflächen vorzunehmen.

Der Erfindung liegt die Aufgabe zugrunde, eine Sprinkleranlage bereitzustellen, die neben den bekannten Funktionen auch Verbesserungen des Raumklimas sowie Schädlingsbekämpfungsmaßnahmen ermöglicht.

Die Erfindung löst diese Aufgabenstellung mit den Merkmalen des Patentanspruches 1. Weitere Ausgestaltungen der Erfindung sind Gegenstand der sich anschließenden Unteransprüche.

Eine Sprinkleranlage, die ein Strömungsleitungssystem mit einem Wasseranschluss, mehreren Fluidleitungen, mindestens eine Pumpe und mehrere, in einem Raum verteilt angeordnete Spritzdüsen zur Zerstäubung einer über die Spritzdüsen abzugebenden Flüssigkeit umfasst, wurde erfindungsgemäß dahingehend weitergebildet, dass mindestens ein Vorratsbehälter mit einem flüssigen Zuschlagstoff zur Herstellung eines Gemisches mit Wasser vorhanden ist, in den saugseitig eine druckseitig mit einem Verteilerrohr gekoppelte Saugleitung mündet, wobei diese Saugleitung eine durch eine elektronische Steuerungsvorrichtung ansteuerbare Dosierpumpe zur Förderung des Zuschlagstoffes aufweist.

Sprinkleranlagen nach der Erfindung können zusätzlich zu ihrer Brandschutzfunktion auch eingesetzt werden, um beispielsweise das Raumklima beziehungsweise insgesamt die Luftqualität in Räumen zu verbessern. Dabei kann zum Beispiel in geringem Umfang reines Wasser versprüht werden, um die Luftfeuchtigkeit zu erhöhen und Staub zu binden. Infolge der sich ausbildenden Verdunstungskälte kann auf diese sehr umweltfreundliche Weise auch die Raumtemperatur abgesenkt werden. Mit dem Versprühen von Wasser lassen sich in vorteilhafter Weise zusätzlich Gerüche binden, sodass insgesamt eine Verbesserung des Raumklimas durch eine derartige Sprinkleranlage möglich wird. Durch die Mischung des zu versprühenden Wassers mit einem Duftstoff kann die Geruchsbindung noch zusätzlich verstärkt sowie insgesamt eine Geruchsverbesserung erreicht werden. Die Behandlung der Raumluft in der zuvor beschriebenen Weise bietet sich jedoch nicht nur in Wohnräumen an. Vielmehr kann eine Sprinkleranlage nach der Erfindung beispielsweise auch bei Messen oder anderen Veranstaltungen zu Desinfektionszwecken zum Einsatz kommen, wo größere Menschenmengen zusammentreffen oder in Ställen, wo naturgemäß eine hohe Staubentwicklung zu verzeichnen ist. Die Verbesserung der Luftqualität ist insbesondere auch bei der Pferdezucht von erheblicher Bedeutung, da in Pferdeställen zum Teil sehr hochwertige Tiere gehalten werden, deren Gesundheit und Wohlbefinden eine maßgebliche Bedeutung für die Leistungsfähigkeit der Pferde hat. Darüber hinaus spielt jedoch auch die Belastung der Tiere durch Insekten eine Rolle, die möglichst reduziert oder verhindert werden sollte. So kann ein Zuschlagstoff für die Gemischbildung mit Wasser auch ein Insektenbekämpfungsmittel sein, dass in dem zuvor erwähnten Stall versprüht wird. In zunehmendem Maße ist jedoch auch eine optimale Desinfektion der Raumluft und der Umgebung von Bedeutung und rückt verstärkt in den Fokus, sodass ein weiterer Zuschlagstoff ein Desinfektionsmittel oder eine Salzlösung sein kann. Die Erfindung besteht folglich aus einer Sprinkleranlage, mit der Flüssigkeit unter Aufbringung eines Hochdrucks zerstäubt und an den zu behandelnden Raum abgegeben wird. Der Flüssigkeit wird dabei in bevorzugter Weise ein beliebiger Zuschlagstoff beigemischt, der den jeweiligen Anforderungen entsprechend austauschbar ist. Die zum Einsatz kommende Pumpe der Sprinkleranlage arbeitet maximal mit einem Druck von etwa 100 bar. Als Spritzdüsen haben sich in besonders bevorzugter Weise Dralldüsen als nützlich erwiesen, da sie geeignet sind, die zu verteilende Flüssigkeit nebelartig zu zerstäuben.

Gemäß einer ersten Ausgestaltung der Erfindung wird vorgeschlagen, dass mehrere Vorratsbehälter mit jeweils unterschiedlichen Zuschlagstoffen vorhanden sind, in die je eine Saugleitung mit je einer durch die elektronische Steuerungsvorrichtung ansteuerbaren Dosierpumpe mündet. Auf diese Weise werden unterschiedliche Zuschlagstoffe bereitgehalten, die je nach Bedarf dem über die Sprinkleranlage zu zerstäubenden Wasser beigemischt werden können. Die Leistung einer derartigen Dosierpumpe kann etwa bei 2,5 bar liegen, sodass bei einer herkömmlichen Wechselspannung von 230 V eine Förderleistung von 5 l/h erreichbar ist.

Um die Füllmenge der einzelnen Vorratsbehälter zuverlässig bestimmen zu können, wird entsprechend einer weiterbildenden Maßnahme der Erfindung vorgeschlagen, dass jeder Vorratsbehälter mit einer Wägeanordnung gekoppelt ist. Die Kopplung mit einer Wägeanordnung kann bedeuten, dass der Vorratsbehälter hängend mit der Wägeanordnung verbunden ist oder auf einer hierfür geeigneten Wägezelle abgestellt wird, die das Gewicht des Vorratsbehälters regelmäßig oder ununterbrochen misst. Dabei ist es von Vorteil, wenn das Gewicht permanent gemessen wird oder bei Bedarf eine Abfrage des Gewichts des Vorratsbehälters erfolgt. Wägezellen sind deshalb von Vorteil, da sie handelsüblich sind und einen sehr genauen Messwert ermöglichen. Zudem kann das ausgegebene Signal elektronisch verarbeitet und somit der elektronischen Steuerungsvorrichtung der erfindungsgemäßen Sprinkleranlage zur weiteren Verarbeitung zugeführt werden. Die Weiterverarbeitung der elektronischen Signale der Wägezellen kann beispielsweise darin bestehen, dass der bevorstehende Leerstand eines Vorratsbehälters mittels einer geeigneten Anzeigevorrichtung signalisiert und damit dem Anwender deutlich gemacht wird.

Zur Verbrauchsmessung beziehungsweise zur Erfassung des Volumens des mittels der Sprinkleranlage versprühten Wassers wird darüber hinaus vorgeschlagen, ein Volumenstrommessgerät vorzusehen, das in Strömungsrichtung betrachtet dem Wasseranschluss folgend in die Fluidleitung integriert ist. Eine alternative oder ergänzende Variante der Erfassung des Verbrauchs frischen Wassers besteht in der Messung des Volumenstroms der zum Einsatz kommenden Pumpe oder sämtlicher Pumpen des Strömungsleitungssystems.

Im Sinne der Erfindung wird das Gemisch aus Wasser und Zuschlagstoff mittels der Sprühdüsen zerstäubt. Um zu vermeiden, dass unerwünscht Fremdkörper in die sehr feinen Düsenöffnungen gelangen, ist es von Vorteil, wenn in Strömungsrichtung betrachtet nach dem Verteilerrohr ein Filter in eine als Versorgungsleitung der Spritzdüsen dienende Fluidleitung integriert ist. Der Filter dient damit der Reinigung des Flüssigkeitsgemischs und sichert auf diese Weise eine lange Lebensdauer der Spritzdüsen.

Bei Erreichen eines definierten Verunreinigungsgrades des Filters muss dieser gereinigt oder gegen einen neuen Filter ausgetauscht werden. Um die Verunreinigung des Filters elektronisch erfassen zu können, wird entsprechend einer Ausgestaltung der Erfindung vorgeschlagen, dass in Flussrichtung betrachtet vor und nach dem Filter ein mit der elektronischen Steuerungsvorrichtung kommunizierender I/V-p-Umformer (Druckmessumformer) vorhanden ist. Mittels der I/V-p-Umformer wird einerseits vor und andererseits nach dem Filter jeweils ein Drucksignal erzeugt und in ein elektronisch verarbeitbares Signal umgewandelt. Die Differenz des erfassten Flüssigkeitsdruckes vor und nach dem Filter erlaubt dabei eine Aussage über den Zustand des Filters.

Zur Erreichung eines gerichteten Fluidstromes innerhalb des Strömungsleitungssystems der Sprinkleranlage wird zumindest in jede Saugleitung ein Einwegventil integriert. Darüber hinaus können einzelne Fluidleitungen zu- oder abgeschaltet werden, was mittels der elektronischen Steuerungsvorrichtung und von dieser ansteuerbarer Mehrwegeventile erfolgt. Durch diese Steuerungsmöglichkeiten können beispielsweise bedarfsgerecht einzelne Vorratsbehälter angesteuert und aus diesen die darin enthaltenen Zuschlagstoffe entnommen und dem Wasser innerhalb der Sprinkleranlage beigemischt werden.

Wie eingangs bereits erwähnt wurde, kann eine erfindungsgemäße Sprinkleranlage bevorzugt auch in Ställen und besonders bevorzugt in Pferdeställen zum Einsatz kommen, um die darin lebenden Tiere optimal zu versorgen. Bei Turnierpferden ist es von besonderer Bedeutung, dass die Vorratsbehälter mit den Zuschlagstoffen nur von autorisierten Personen ausgetauscht beziehungsweise aufgefüllt werden, wenn deren Inhalt verbraucht ist. Aus diesem Grund wird vorgeschlagen, dass die Vorratsbehälter vor unberechtigtem Zugriff geschützt untergebracht sind und ein Zugriff nur per PIN-Code, per Fingerabdruckscanner oder Gesichtsscanner möglich ist.

Ein weiterer, sehr wesentlicher Vorteil der Erfindung ist darüber hinaus darin zu sehen, dass die elektronische Steuerungsvorrichtung derart ausgelegt ist, dass ihre Steuerung durch ein Computerprogramm erfolgt, dessen Steuerungssignale drahtlos von einer externen Steuerungssoftware (App) an die elektronische Steuerungseinheit übertragbar sind. So ist es möglich, die Sprinkleranlage über ein externes Endgerät, also beispielsweise über ein Mobiltelefon oder ein Notebook anzusteuern. Die Bedienperson muss folglich nicht in jedem Fall persönlich anwesend sein, um in das System eingreifen und dieses beeinflussen zu können.

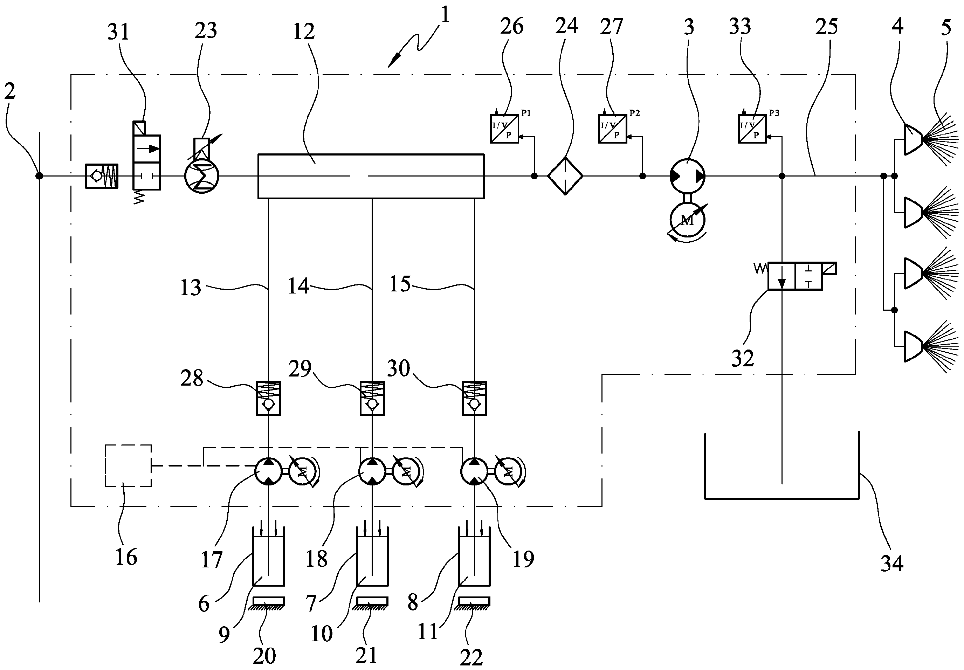

Die Erfindung wird nachfolgend anhand der beigefügten Zeichnung näher erläutert. Das gezeigte Ausführungsbeispiel stellt dabei keine Einschränkung auf die dargestellte Variante dar, sondern dient lediglich der Erläuterung eines Prinzips der Erfindung.

Um die erfindungsgemäße Funktionsweise veranschaulichen zu können, ist in der Figur nur eine stark vereinfachte Prinzipdarstellung gezeigt, bei der auf die für die Erfindung nicht wesentlichen Bauteile verzichtet wurde. Dies bedeutet jedoch nicht, dass derartige Bauteile bei einer erfindungsgemäßen Lösung nicht vorhanden sind.

Die einzige Figur zeigt eine Sprinkleranlage 1, die, ausgehend von einem Wasseranschluss 2, ein Strömungsleitungssystem darstellt, welches zahlreiche Fluidleitungen, eine Pumpe 3 und mehrere in einem Raum verteilt angeordnete Spritzdüsen 4 zur zerstäubten Abgabe einer Flüssigkeit 5 umfasst. Bei der in der Figur dargestellten Ausführungsvariante einer Sprinkleranlage 1 sind insgesamt drei Vorratsbehälter 6, 7, 8 vorhanden, wobei jeder der Vorratsbehälter 6, 7, 8 je einen Zuschlagstoff 9, 10, 11 aufnimmt. Bei den Zuschlagstoffen 9, 10, 11 handelt es sich beispielsweise um Duftstoffe, um Insektenbekämpfungsmittel oder um eine salzhaltige Lauge, sodass durch Vermischung eines Zuschlagstoffes 9, 10, 11 mit dem im Strömungsleitungssystem der Sprinkleranlage 1 vorhandenen Wasser ein Flüssigkeitsgemisch 5 gebildet wird, das für ein verbessertes Raumklima oder zum Schutz vor Insekten in einen Innenraum nutzbar ist. In einem Stall für Tiere kann auf diese Art und Weise zum Beispiel in zerstäubter Form ein Insektenbekämpfungsmittel versprüht werden, was dazu führt, dass das Leid der Tiere durch die Insekten reduziert oder vermieden wird. Für ein besonderes Wohlbefinden ist es zudem möglich, Duftstoffe oder salzhaltige Lösungen zu vernebeln. Diese Zuschlagstoffe 9, 10, 11 werden, wie bereits ausgeführt, in den Vorratsbehältern 6, 7, 8 aufbewahrt und können wahlweise zum Einsatz kommen. Zur Entnahme der einzelnen Zuschlagstoffe 9, 10, 11 aus den Vorratsbehälter 6, 7, 8 weist jede vorhandene Saugleitung 13, 14, 15 je eine Dosierpumpe 17, 18, 19 auf. Jede der Dosierpumpen 17, 18, 19 wird über eine in der Figur lediglich andeutungsweise dargestellte, elektronische Steuerungsvorrichtung 16 angesteuert, sodass darüber die Auswahl des einzusetzenden Zuschlagstoffes 9, 10, 11 erfolgen kann. Jeder der Vorratsbehälter 6, 7, 8 steht in einer Wirkverbindung mit einer Wägeanordnung, bei der es sich hier um je eine unter dem Vorratsbehälter 6, 7, 8 angeordnete Wägezelle 20, 21, 22 handelt. Die Wägezellen 20, 21, 22 erzeugen in an sich bekannterweise ein gewichtsproportionales, elektronisches Signal, das in der elektronischen Steuerungsvorrichtung 16 verarbeitet wird, sodass dadurch der Füllstand der einzelnen Vorratsbehälter 6, 7, 8 bestimmt und zur Anzeige gebracht werden kann. Die in die Vorratsbehälter 6, 7, 8 mündenden Saugleitungen 13, 14, 15 gehen auf ihrer dem Vorratsbehälter 6, 7, 8 abgewandten Seite in ein Verteilerrohr 12 über, das eine Verbindung zwischen dem Wasseranschluss 2 und den Spritzdüsen 4 mit den Saugleitungen 13, 14, 15 der Sprinkleranlage 1 bildet. Zur Erzielung einer gerichteten Strömung innerhalb der Saugleitungen 13, 14, 15 dienen jeweils in die Saugleitungen 13, 14, 15 integrierte Einwegventile 28, 29, 30. In Strömungsrichtung betrachtet weist das Strömungsleitungssystem der Sprinkleranlage 1, ausgehend vom Wasseranschluss 2, ferner ein Mehrwegventil 31 auf, dem sich ein Volumenstrommessgerät 23 anschließt. Mit dem Volumenstrommessgerät 23 wird das von der Pumpe 3 geförderte Wasser gemessen und dessen Volumen auf diese Weise erfasst. Nachdem sich anschließenden Verteilerrohr 12 folgt in dem Strömungsleitungssystem ein Filter 24 dem jeweils ein I/V-p-Umformer 26 vorgeschaltet und ein I/V-p-Umformer 27 nachgeschaltet ist. Aus der Differenz der auf diese Weise ermittelten Drücke vor und nach dem Filter 24 lässt sich der Zustand des Filters ermitteln, sodass dadurch ein zuverlässiger Hinweis auf eine erforderliche Reinigung beziehungsweise auf den erforderlichen Austausch des Filters 24 gegeben werden kann. Zur Anzeige der einzelnen Werte weist die Sprinkleranlage 1 eine in der Figur nicht gezeigte Anzeigevorrichtung auf, bei der es sich beispielsweise um ein Display oder eine elektronische Informationstafel handeln kann. Übersteigt der Druck in der Versorgungsleitung 25 einen zulässigen Höchstwert, so sorgt ein weiterer I/V-p-Umformer 33 dafür, dass ein Druckausgleich erfolgen kann. Hierzu dient ein Mehrwegventil 32, dass in einer Ablaufleistung integriert ist, sodass Flüssigkeit in ein hierfür bereitstehendes Auffangbecken 34 abgeführt werden kann.

### BEZUGSZEICHENLISTE:

- 1: Sprinkleranlage
- 2: Wasseranschluss
- 3: Pumpe
- 4: Spritzdüsen
- 5: Flüssigkeit
- 6: Vorratsbehälter
- 7: Vorratsbehälter
- 8: Vorratsbehälter
- 9: Zuschlagstoff
- 10: Zuschlagstoff
- 11: Zuschlagstoff
- 12: Verteilerrohr
- 13: Saugleitung
- 14: Saugleitung
- 15: Saugleitung
- 16: elektronische Steuerungsvorrichtung
- 17: Dosierpumpe
- 18: Dosierpumpe
- 19: Dosierpumpe
- 20: Wägeanordnung
- 21: Wägeanordnung
- 22: Wägeanordnung
- 23: Volumenstrommessgerät
- 24: Filter
- 25: Versorgungsleitung
- 26: I/V-p-Umformer
- 27: I/V-p-Umformer
- 28: Einwegventil
- 29: Einwegventil
- 30: Einwegventil
- 31: Mehrwegventil
- 32: Mehrwegventil
- 33: I/V-p-Umformer
- 34: Auffangbecken

## Patentansprüche

1. Sprinkleranlage (1), umfassend ein Strömungsleitungssystem mit einem Wasseranschluss (2), mehreren Fluidleitungen, mindestens einer Pumpe (3) und mit mehreren, in einem Raum verteilt angeordneten Spritzdüsen (4) zur Zerstäubung einer über die Spritzdüsen (4) abzugebenden Flüssigkeit (5),
**dadurch gekennzeichnet, dass**
mindestens ein Vorratsbehälter (6, 7, 8) mit einem flüssigen Zuschlagstoff (9, 10, 11) zur Herstellung eines Gemisches mit Wasser vorhanden ist, in den saugseitig eine druckseitig mit einem Verteilerrohr (12) gekoppelte Saugleitung (13, 14, 15) mündet, wobei diese Saugleitung (13, 14, 15) eine durch eine elektronische Steuerungsvorrichtung (16) ansteuerbare Dosierpumpe (17, 18, 19) zur Förderung des Zuschlagstoffes (9, 10, 11) aufweist.

2. Sprinkleranlage nach Anspruch 1,
**dadurch gekennzeichnet, dass**
mehrere Vorratsbehälter (6, 7, 8) mit unterschiedlichen Zuschlagstoffen (9, 10, 11) vorhanden sind, in die jeweils eine Saugleitung (13, 14, 15) mit je einer durch die elektronische Steuerungsvorrichtung (16) ansteuerbaren Dosierpumpe (17, 18, 19) mündet.

3. Sprinkleranlage nach einem der vorstehend genannten Ansprüche,
**dadurch gekennzeichnet, dass**
jeder Vorratsbehälter (6, 7, 8) zur Erfassung seines Füllstandes mit einer Wägeanordnung (20, 21, 22) gekoppelt ist.

4. Sprinkleranlage nach Anspruch 3,
**dadurch gekennzeichnet, dass**
jede Wägeanordnung (20, 21, 22) eine unter dem Vorratsbehälter angeordnete Wägezelle ist.

5. Sprinkleranlage nach einem der vorstehend genannten Ansprüche, **dadurch gekennzeichnet, dass**
in Strömungsrichtung betrachtet dem Wasseranschluss (2) ein in die Fluidleitung integriertes Volumenstrommessgerät (23) nachgeordnet ist.

6. Sprinkleranlage nach einem der vorstehend genannten Ansprüche, **dadurch gekennzeichnet, dass**
in Strömungsrichtung betrachtet nach dem Verteilerrohr (12) ein Filter (24) in eine als Versorgungsleitung (25) der Spritzdüsen (4) dienende Fluidleitung integriert ist.

7. Sprinkleranlage nach Anspruch 6,
**dadurch gekennzeichnet, dass**
zur Erfassung des Filterzustandes in Flussrichtung betrachtet vor und nach dem Filter je ein mit der elektronischen Steuerungsvorrichtung (16) kommunizierender I/V-p-Umformer (26 und 27) vorhanden ist.

8. Sprinkleranlage nach einem der vorstehend genannten Ansprüche, **dadurch gekennzeichnet, dass**
in jeder Saugleitung (13, 14, 15) je ein Einwegventil (28, 29, 30) vorhanden ist und die Zu- oder Abschaltung einzelner Fluidleitungen mittels vorhandener, mittels der elektronischen Steuerungsvorrichtung (16) ansteuerbarer Mehrwegeventile (31, 32) erfolgt.

9. Sprinkleranlage nach einem der vorstehend genannten Ansprüche, **dadurch gekennzeichnet, dass**
die Vorratsbehälter (6, 7, 8) vor unberechtigtem Zugriff geschützt untergebracht sind und ein Zugriff nur per PIN-Code, per Fingerabdruckscanner oder Gesichtsscanner möglich ist.

10. Sprinkleranlage nach einem der vorstehend genannten Ansprüche, **dadurch gekennzeichnet, dass**
die elektronische Steuerungsvorrichtung (16) derart ausgelegt ist, dass ihre Steuerung durch ein Computerprogramm erfolgt, dessen Steuerungssignale drahtlos von einer externen Steuerungssoftware (App) an die elektronische Steuerungseinheit übertragbar sind.
